# FASCICULE DE BREVET EUROPEEN

(11) **EP 2 111 362 B1**
(45) Date de publication et mention de la délivrance du brevet: **17.08.2011**
(21) Numéro de dépôt: 08761734.6
(22) Date de dépôt: 03.01.2008
(51) Int. Cl.: B65D 50/04, A61F 9/00, B65D 47/18

(54) **ENSEMBLE DE CONDITIONNEMENT ET DE DISTRIBUTION DE COLLYRE**
ANORDNUNG ZUM ENTHALTEN UND AUSGEBEN VON AUGENTROPFEN
ASSEMBLY FOR CONDITIONING AND DISPENSING EYE DROPS

(30) Priorité: 12.01.2007 FR 0700245
(43) Date de publication de la demande: 28.10.2009
(73) Titulaire: Rexam Pharma, 76550 Offranville (FR)
(72) Inventeur: GREVIN, Guillaume, 38080 L'Isle-d'Abeau (FR)
(74) Mandataire: Blot, Philippe Robert Emile
(86) Numéro de dépôt international: PCT/FR2008/000010
(87) Numéro de publication internationale: WO 2008/099079

(56) Documents cités:
- EP-A- 0 111 419
- EP-A- 0 343 778
- DE-U1- 9 312 423
- FR-A- 2 414 005
- GB-A- 2 256 638
- US-A1- 2006 111 680

## Description

La présente invention concerne un ensemble de conditionnement et de distribution de collyre, du type comprenant :
- un récipient destiné à contenir du liquide, le récipient possédant un col ;
- un embout compte-gouttes de distribution disposé à l'extrémité du col, l'embout et le récipient permettant la distribution de liquide goutte par goutte par pincement du récipient entre les doigts d'une main :
- un bouchon de fermeture du récipient prévu pour être vissé sur le col ; et
- un témoin de première ouverture sous la forme d'une ceinture d'inviolabilité reliée au bouchon par une région frangible destinée à se rompre lors de la première ouverture du bouchon. Un tel ensemble est décrit dans US 2006/0111680 A1.

Dans un tel ensemble de conditionnement et de distribution, les risques qu'un enfant utilise le liquide de façon inappropriée sans le contrôle d'un adulte, par exemple en l'ingérant, sont très limités du fait de la présence de l'embout compte-gouttes, qui limite les quantités de liquide distribuées. En outre, les liquides médicaux ophtalmiques comme par exemple les collyres, destinés à être déposés sur l'oeil, sont considérés généralement comme peu dangereux.

Néanmoins, Il est souhaitable de limiter les risques d'utilisation inappropriée.

Un but de l'invention est de fournir un ensemble de conditionnement et de distribution d'un liquide médical ophtalmique permettant de limiter les risques d'utilisation inappropriée, en particulier par un enfant.

A cet effet, l'invention propose un ensemble de conditionnement et de distribution de collyre du type précité, caractérisé en ce que le bouchon et le col possèdent des moyens de sécurité interdisant le dévissage du bouchon par la seule application d'un couple de dévissage, et autorisant le dévissage du bouchon lorsqu'une action supplémentaire est exercée conjointement au couple de dévissage du bouchon.

Selon d'autres modes de réalisation, l'ensemble de conditionnement et de distribution comprend une ou plusieurs des caractéristiques suivantes, prise(s) isolément ou selon toutes les combinaisons techniquement possibles :
- le récipient étant réalisé dans un matériau ou un mélange de matériaux possédant un module d'Young inférieur au module d'Young du matériau ou du mélange de matériaux dans lequel est réalisé le bouchon ;
- le récipient et le bouchon sont chacun réalisé à partir d'un matériau ou d'un mélange de matériaux choisi(s) parmi les matériaux suivants : le polyéthylène basse densité (PEBD), le polyéthylène moyenne densité (PEMD), le polyéthylène haute densité (PEHD), le polypropylène et le polyethyléne-téréphtalate (PET) ;
- le récipient et le bouchon sont réalisés à partir de polyéthylène basse densité (PEBD) et de polyéthylène haute densité (PEHD), le bouchon possédant une proportion en masse de polyéthylène haute densité (PEHD) plus importante que le récipient ;
- le récipient est constitué uniquement de polyéthylène basse densité (PERD), et le bouchon comprend au moins 20% en masse de polyéthylène haute densité (PEHD), le reste étant constitué de polyéthylène basse densité (PEBD) ;
- le récipient possède une contenance inférieure à 30 ml, de préférence inférieure à 20 ml ;
- les moyens de sécurité permettent le dévissage du bouchon si le bouchon est pincé pendant le dévissage ;
- les moyens de sécurité possèdent des moyens de blocage unidirectionnel en rotation du bouchon par rapport au col, les moyens de blocage unidirectionnel étant débrayables par pincement du bouchon ;
- le bouchon possède une première jupe externe coaxiale à et entourant une deuxième jupe portant un filetage de vissage du bouchon sur le col, les moyens de blocage possédant au moins un relief intérieur de la première jupe et au moins un relief extérieur du col complémentaires, pouvant être dégagés l'un de l'autre par pincement de la première jupe sur deux zones décalées angulairement par rapport au ou à chaque relief intérieur ;
- les moyens de blocage possèdent deux reliefs intérieurs disposés sur la jupe, diamétralement opposés et décalés angulairement de 90° par rapport aux zones d'appui, le col comprenant deux reliefs extérieurs diamétralement opposés ;
- le ou chaque relief extérieur du col est formé par un ergot en saillie radialement vers l'extérieur du col l'ergot possédant une surface de blocage radiale, une surface de glissement inclinée dans son ensemble par rapport à la surface de blocage, et une surface de transition s'étendant entre la surface de blocage et la surface de glissement, la surface de transition étant sensiblement perpendiculaire à la surface de blocage ;
- le col possède une couronne annulaire de renfort s'étendant en saillie vers l'extérieur du col et passant par les reliefs extérieurs pour les rigidifier ;
- le bouchon possède un organe de fermeture étanché de l'embout, obturant l'embout au moins sur la course du bouchon pendant laquelle les moyens de sécurité sont opérationnels pour assurer l'étanchéité et la stérilité à l'intérieur du récipient ;
- le récipient contient un liquide médical ophtalmique du type collyre.

L'invention et ses avantages seront mieux compris à la lecture de la description qui va suivre, donnée uniquement à titre d'exemple, et faite en se référant aux dessins annexés, sur lesquelles :
- la figure 1 est une vue de côté en élévation d'un ensemble de conditionnement et de distribution conforme à l'invention ;
- la figure 2 est une vue en coupe selon II-II de l'ensemble de la figure 1 ;
- la figure 3 est une vue en perspective de l'ensemble de conditionnement et de distribution de la figure 1, avant montage d'un bouchon de l'ensemble ;
- la figure 4 est une vue en section selon IV-IV de la figure 1, illustrant le bouchon dans une configuration au repos ;
- la figure 5 est une vue analogue à celle de la figure 4, illustrant le bouchon dans une configuration déformée par pincement ; et
- la figure 6 est une vue en section selon VI-VI de la figure 1.

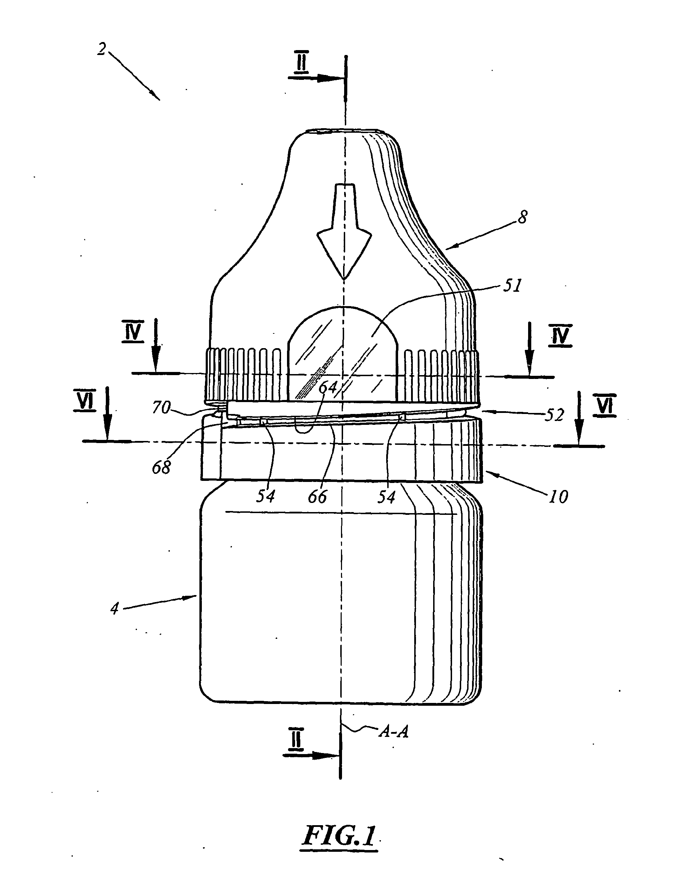

Les figures 1 à 3 illustrent un ensemble 2 de conditionnement et de distribution d'un collyre sous forme d'une solution aqueuse.

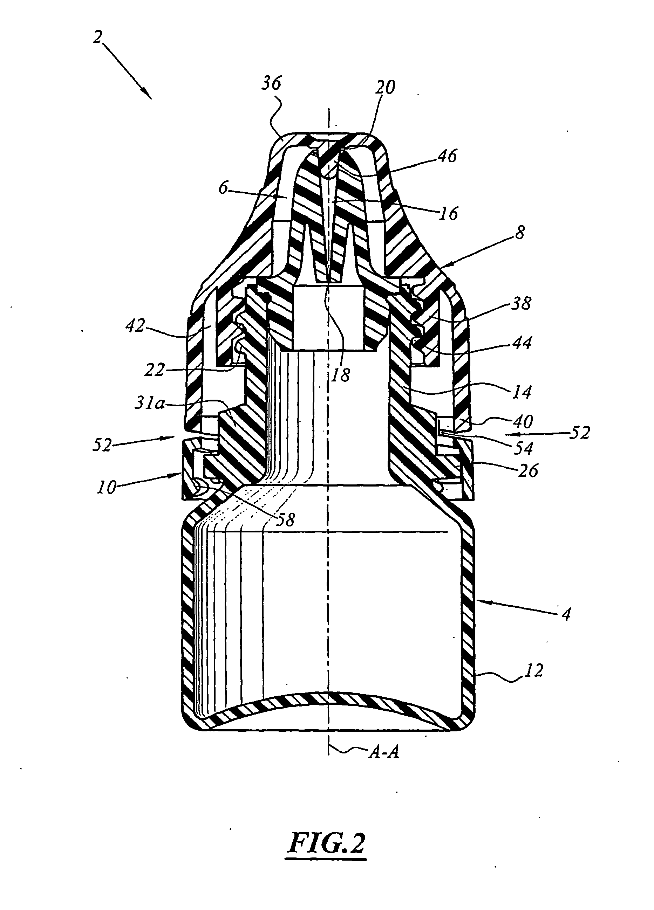

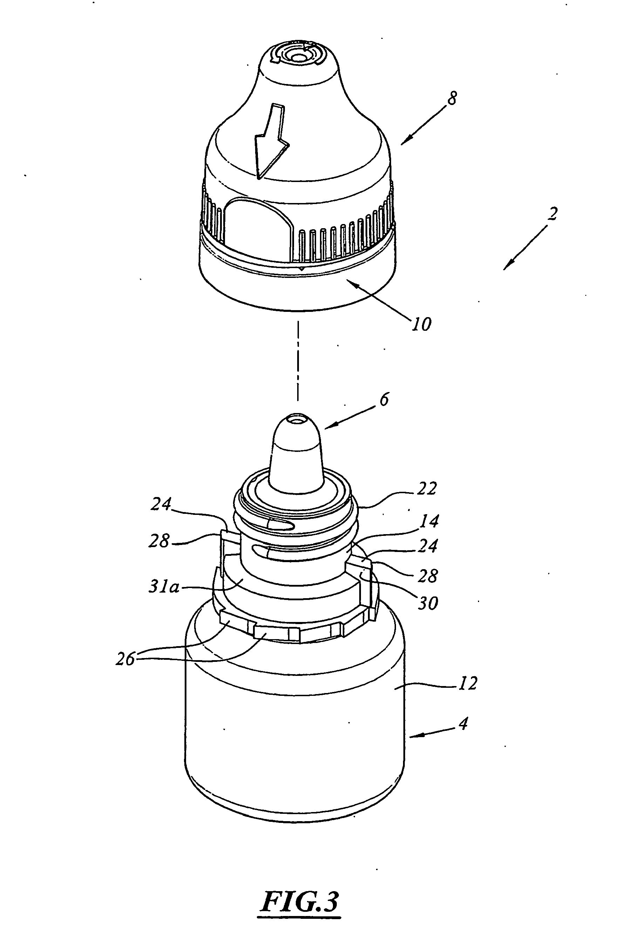

L'ensemble 2 comprend un récipient 4 destiné à contenir le collyre, un embout 6 (figure 2 et 3) compte-gouttes, un bouchon 8 de fermeture du récipient 4, et une ceinture ou bague d'inviolabilité 10 formant témoin de première ouverture de l'ensemble 2.

Le récipient 4 est dans l'exemple illustré un flacon en matière plastique, contenant le collyre qui n'a pas été représenté sur les figures.

Tel que représenté sur les figure 2 et 3, le récipient 4 possède un corps 12 creux prolongé par un col 14, qui s'étend selon un axe longitudinal A-A.

Les parois du corps 12 sont déformables élastiquement par pincement pour provoquer une diminution du volume interne du corps 12, et l'expulsion du collyre au travers du col 14.

Tel que représenté sur les figures 2 et 3, l'embout 6 est enfiché dans le col 14. L'embout 6 possède un conduit 16 pour le passage du collyre, s'élargissant progressivement entre un orifice amont 18 (en bas sur la figure 2) et un orifice aval 20.

Le conduit 16 permet de distribuer le collyre goutte par goutte, avec une taille de goutte calibrée, lorsque le corps 12 est pincé par l'utilisateur entre ses doigts.

Le col 14 possède plusieurs reliefs extérieurs comprenant, depuis l'extrémité du col 14 opposée au corps 12 vers ce dernier, un filetage 22 supérieur, des ergots 24 (figure 3) intermédiaires, et des dents 26 inférieures.

Comme représenté sur les figures 3 à 5, le col 14 comprend deux ergots 24 diamétralement opposées faisant saillie radialement vers l'extérieur du col 14.

Chaque ergot 24 s'étend radialement vers l'extérieur à partir du col 14, et possède à son extrémité extérieure une surface de glissement 28 et une surface de blocage 30 sensiblement radiale.

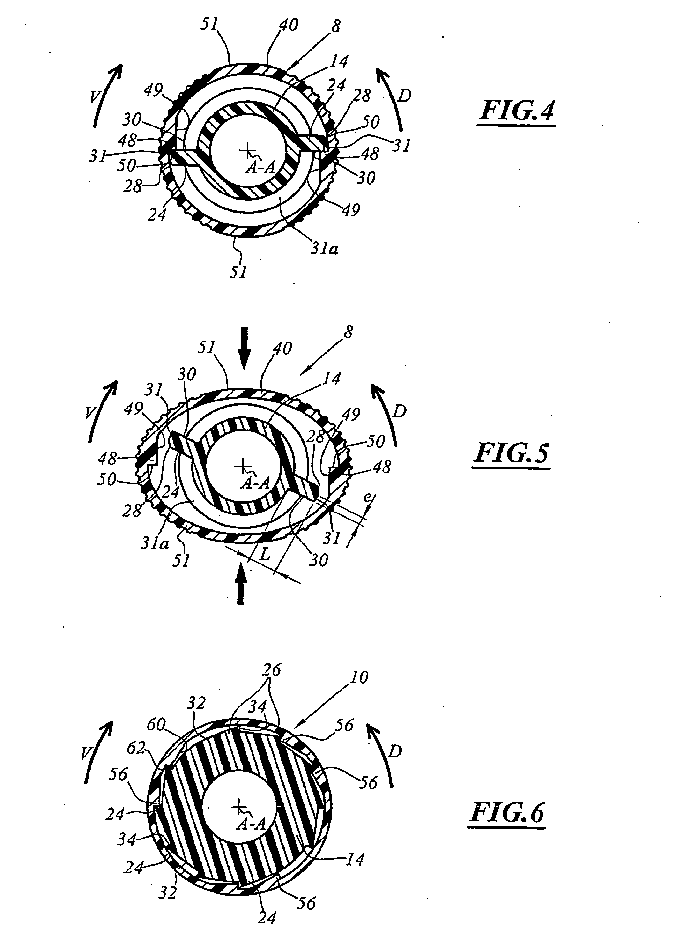

Chaque ergot 24 possède une surface de transition 31 (figure 4 et 5) s'étendant entre la surface de glissement 28 et la surface de blocage 30. La surface de transition 31 est sensiblement perpendiculaire à la surface de blocage 30. La surface de transition assure que l'ergot 24 possède une épaisseur minimale à son extrémité extérieure pour assurer une rigidité suffisante à l'ergot 24.

Le col 14 possède une couronne 31a annulaire de renfort s'étendant en saillie radialement vers l'extérieur à partir du col 14, à hauteur des ergots 24.

Ainsi, la couronne 31a rigidifie chaque ergot 24 en flexion dans le sens circonférentiel.

La couronne 31a possède une hauteur, suivant l'axe A-A, inférieure à celle des ergots 24.

La couronne 31a possède un diamètre extérieur inférieur au diamètre du cercle imaginaire passant par les extrémités extérieures des ergots 24, pour ne pas empêcher lesdites extrémités extérieures de venir en prises avec des reliefs complémentaires du bouchon 8, comme cela sera décrit par la suite.

On notera que les ergots 24 ne sont pas visibles sur la figure 2 car le plan de coupe ne passe pas par les ergots 24. Le plan de coupe de la figure 3 passe au-dessus de la couronne 31a.

Comme représenté sur la figure 6, le col 14 possède une pluralité de dents 26 réparties autour du col 14 et faisant saillie vers l'extérieur. Les dents 26 sont ici au nombre de dix, et répartis en deux séries de cinq dents, les deux séries étant diamétralement opposées.

Chaque dent 26 s'étend radialement vers l'extérieur et possède une surface de glissement 32 et une surface de blocage 34. Les dents 26 adjacentes définissent entre elles des crans.

Les dents 26 s'étendent à partir de l'extrémité inférieure de la couronne 31a (figure 3).

Tel que représenté sur la figure 2, le bouchon 8 possède un fond 36 et deux jupes 38, 40 concentriques s'étendant longitudinalement vers le bas à partir du fond 36.

La jupe 40 externe entoure la jupe 38 interne, et est plus longue que la jupe 38. Un espace 42 annulaire est ménagé entre les jupes 38, 40. La jupe 40 est déformable élastiquement par pincement.

La jupe 38 porte un filetage 44 intérieur complémentaire du filetage 22, et permettant de visser le bouchon 8 sur le col 14.

Le fond 36 porte un relief intérieur sous la forme d'un téton 46 central d'obturation du conduit 16. Ce téton 46 fait saillie vers le bas à partir du fond 36.

Comme représenté sur la figure 3, la jupe 40 porte deux saillies 48 internes diamétralement opposées, prévues pour coopérer avec les ergots 24.

Chaque saillie 48 possède une surface de glissement 49 et une surface de blocage 50 sensiblement radiale.

La jupe 40 possède des zones 51 externes d'appui pour les doigts, diamétralement opposées, et décalées de sensiblement 90° autour de l'axe A-A par rapport aux saillies 48.

Dans une configuration au repos de la jupe 40 (figure 4), la jupe 40 est sensiblement circulaire, et les saillies 48 interfèrent avec les ergots 24 du col 14 en cas de rotation du bouchon 8 par rapport au col 14.

Les ergots 24 et les saillies 48 sont orientés de façon qu'en cas de rotation du bouchon 8 par rapport au col 14 dans le sens horaire de vissage matérialisé par une flèche V sur la figure 4, les saillies 48 et les ergots 24 viennent en contact par leurs surfaces de glissement 28, 49. L'application d'un couple de rotation autour de l'axe A-A suffisant sur le bouchon 8 provoque, du fait de l'inclinaison des surfaces de glissement 28, 49, une déformation de la jupe 40 permettant aux saillies 48 de passer les ergots 24 sans empêcher la rotation du bouchon 8.

En cas de rotation du bouchon 8 par rapport au col 14 dans le sens anti-horaire de dévissage matérialisé par une flèche D sur la figure 4, les saillies 48 et les ergots 24 vienne en appui mutuel par leur surfaces de blocages 50, 30 et interdisent la rotation du bouchon 8 par rapport au col 14.

Comme illustré sur la figure 5, dans une configuration déformée de la jupe 40, par pincement exercée sur les zones 51, la jupe 40 adopte une forme sensiblement ovale ou elliptique, de sorte que les saillies 48 sont écartées par rapport à la configuration au repos de la jupe 40. Les saillies 48 sont dégagés des ergots 24, et le dévissage du bouchon 8 est possible.

Les saillies 48 portées par la jupe 40 déformable élastiquement et les ergots 24 portés par le col 14 forment des moyens de blocage unidirectionnel en rotation du bouchon 8 par rapport au col 14 aptes à interdire le dévissage du bouchon 8, qui sont débrayables par pincement de la jupe 40, pour permettre le dévissage du bouchon 8.

Tel que représentée sur les figure 1, 2 et 6, la bague 10 est venue de matière avec le bouchon 8, et reliée à celui-ci par une région 52 frangible, formée par des pontets 54 frangibles (figure 1).

La bague 10 possède des reliefs intérieurs, comprenant des dents 56 (figure 6) et des nervures 58 (figure 2).

Les nervures 58 sont disposées longitudinalement sous les dents 56. Les nervures 58 sont ici au nombre de trois et régulièrement réparties autour de l'axe A-A. Chaque nervure 58 s'étend en arc de cercle autour de l'axe A-A, et présente une forme bombée.

Comme cela est visible sur la figure 6, les dents 56 sont complémentaires des dents 26. Chaque dent 56 possède une surface de glissement 60 et une surface de blocage 62.

La bague 10 possède six dents 56, réparties en trois paires décalées de 120° autour de l'axe A-A.

Les dents 56 et 26 sont orientées de façon qu'en cas de rotation de la bague 10 par rapport au col 14 dans le sens anti-horaire de dévissage matérialisé par la flèche D sur la figure 6, les dents 56 et 26 viennent en contact par leurs surfaces de blocages 62, 34 et interdisent la rotation de la bague 10 par rapport au col 14.

En cas de rotation en sens inverse, les dents 56 et 26 viennent en contact par leurs surfaces de glissement, et un couple de rotation suffisant permet de faire tourner la bague 10 par rapport au col 14.

Les dents 56 et 26 forment donc des moyens de blocage unidirectionnel en rotation de la bague 10 par rapport au col 14.

Les répartitions des dents 26 et 56 assurent qu'il existe des dents 26 en prise avec des dents 56 lorsque la bague 10 est en position sur le col 14.

Le diamètre de la bague 10 est tel que la bague 10 peut se déplacer le long du col 14 sans interférer radialement avec les ergots 24.

Tel que représentées sur la figure 1, la jupe 40 et la bague 10 possèdent sur leurs bords 64, 66 adjacents des crans 68, 70 complémentaires de blocage en rotation du bouchon 8 par rapport à la bague 10 dans le sens du vissage.

La jupe 40 et la bague 10 comprennent chacun deux crans 68, respectivement 70, diamétralement opposés.

L'ensemble 2 est livré en vue du conditionnement avec l'embout 6, le bouchon 8 et la bague 10 séparés du récipient 4.

Pour le conditionnement du collyre, le récipient 4 est rempli en collyre, puis l'embout 6 est enfiché dans le col 14, et le bouchon 8, portant la bague 10, est vissé sur le col 14.

Lors du vissage du bouchon 8, les filetages 22 et 44 coopèrent pour provoquer la descente du bouchon 8 et de la bague 10 le long du col 14.

La bague 10 descend le long du col 14 sans interférer avec les ergots 24. Lorsque les nervures 58 rencontrent les dents 26, il faut exercer un couple de rotation plus important, et une poussée vers le bas sur le bouchon 8.

Le bouchon 8 pousse la bague 10 vers le bas, et entraîne la bague 10 en rotation par la coopération des crans 68, 70.

Du fait de la poussée exercée sur la bague 10, les dents 26 provoquent, grâce à la forme bombée des nervures 58, une légère expansion élastique de la bague 10, qui va permettre le passage des nervures 58 autour des dents 26.

Du fait de la coopération des crans 68, 70, les pontets 54 ne sont sensiblement pas sollicités circonférentiellement lors du premier vissage du bouchon 8, et la bague 10 est poussée vers le bas le long du col 14 sans rupture des pontets 54.

Le vissage du bouchon 8 est poursuivi jusqu'à ce que les nervures 58 soient passées sous les dents 26, et que les dents 26 soient engagées dans les crans 56.

Lors du vissage du bouchon 8, les saillies 48 et les ergots 24 viennent en contact par leur surface de glissement 49 et 28 ce qui provoque une déformation de la jupe 40 permettant le passage des saillies 48 sur les ergots 24 sans empêcher la rotation du bouchon 8 par rapport au col 14.

A la fin du vissage du bouchon 8, le téton 46 est inséré dans le conduit 16 et assure la fermeture étanche du conduit 16.

L'ensemble 2 est alors dans sa configuration assemblée avant utilisation, comme illustré sur les figures 1 à 3 et 6.

Lors de la première utilisation et de toutes les utilisations suivantes, si l'utilisateur exerce uniquement sur le bouchon 8 un couple de dévissage autour de l'axe A-A, les saillies 48 et ergots 24 interdisent la rotation du bouchon 8 et son dévissage.

Pour dévisser le bouchon 8, l'utilisateur doit exercer, simultanément au couple de dévissage, une action de pincement de la jupe 40 sur les zones 51. Comme décrit précédemment, cette action de pincement déforme la jupe 40 se sorte que les saillies 48 sont dégagées des ergots 24, autorisant alors le dévissage du bouchon 8.

Le pincement doit être exercé au moins dans une première phase du dévissage du bouchon 8, jusqu'à ce que les saillies 48 soient décalées longitudinalement par rapport aux ergots 24.

Lors de la première ouverture de l'ensemble 2, l'utilisateur pince la jupe 40 et exerce un couple de dévissage du bouchon 8. Les filetages 22 et 44 coopèrent pour provoquer le déplacement du bouchon le long du col 14 vers le haut.

Le bouchon 8 est lié à la bague 10 par les pontets 54, de sorte que le bouchon 8 entraîne la bague 10. Les dents 26 et 56 empêchent la rotation de la bague 10 par rapport au col 14. Le dévissage du bouchon 8 se poursuivant, les pontets 54 sont étirés, ce qui provoque rapidement leur rupture.

Le bouchon 8 est alors séparé de la bague 10, et le dévissage du bouchon 8 peut être poursuivi librement jusqu'à son dégagement complet du col 14.

Les nervures 58 retiennent la bague 10 sur le col 14 lorsque le bouchon 8 est retiré, pour éviter la chute de la bague 10, notamment lorsque le récipient 4 est retourné pour la distribution de gouttes dans l'oeil.

Entre deux utilisations, le bouchon 8 est refermé en exerçant simplement un couple de vissage, car ce sens de rotation est autorisé par les saillies 48 et les ergots 24.

Le pas de vis des filetages 22 et 44 et la hauteur des saillies 48 et des ergots 24 sont de préférences choisis de sorte que, lors du dévissage du bouchon 8, chaque saillies 48 passe en regard et à hauteur d'au moins deux ergots 24, pour limiter encore le risque d'ouverture du récipient 4 par un enfant, si par hasard l'enfant parvenant à dévisser partiellement le bouchon en passant un ergot 24.

La hauteur du téton 46 est de préférence choisie pour que le téton 46 obture le conduit 16 de façon étanche au moins sur la course axiale du bouchon pendant laquelle les moyens de sécurité sont opérationnels, c'est-à-dire jusqu'à ce que les saillies 48 soient passées au-dessus des ergots 24.

Ainsi, le récipient 4 reste fermé jusqu'à ce que les moyens de sécurité soient inopérants, ce qui limite le risque que du liquide soit expulsé avant le dévissage complet et volontaire du bouchon 8, et assure l'étanchéité et la stérilité du récipient 4.

Pour la distribution de collyre, l'utilisateur saisit le récipient 4 ouvert, généralement entre le pouce et l'index d'une même main, retourne le récipient 4, et pince le récipient 4 entre ses doigts pour faire sortir un nombre de gouttes déterminé.

Le récipient 4, l'embout 6 et le bouchon 8 doivent respecter certaines contraintes liées au conditionnement des collyres, à la fonction de sécurité enfant, et à la distribution de gouttes calibrées de liquide par pincement du récipient 4 entre les doigts d'une main.

Le récipient 4 et l'embout 6 doivent permettre le pincement entre les doigts. La force applicable par pincement par une personne, notamment une personne âgée, et de l'ordre de 35N à 40N.

Pour cela le récipient doit être suffisamment souple. Cependant, le récipient 4 possède des petites dimensions qui nuisent à sa souplesse. En effet, du fait de leur posologie, les collyres sont conditionnés dans des récipients 4 de petite contenance. Une posologie classique consiste à appliquer deux gouttes de collyre, une ou deux fois par jour, pendant une à deux semaines. La taille d'une goutte de collyre est de l'ordre de 40 µl.

Ainsi, le récipient 4 possède une contenance inférieure à 30 ml, de préférence inférieure à 20 ml. Il est par exemple possible de prévoir une gamme de récipients ayant des contenances de 5ml, 10ml et 15ml.

Les petites dimensions du récipient 4 qui en découlent nuisent à sa souplesse. Le corps 14 du récipient 4 possède typiquement un diamètre de l'ordre de 25 mm, et une hauteur de l'ordre de 15 à 50 mm selon sa contenance.

L'embout 6 possède certaines dimensions rendant sa fabrication compatible avec le contrôle du calibrage des gouttes. Les dimensions du col 14 doivent correspondre à celle de l'embout 6 pour assurer une fermeture étanche du col 14 par l'embout 6, à la périphérie de l'embout 6. Il est en outre préférable de pouvoir utiliser un embout déjà existant, possédant des dimensions prédéterminées.

Par ailleurs, pour assurer la fonction de sécurité enfant, le col 14, et en particulier les ergots 24, et le bouchon 8, et en particulier la jupe 40, doivent posséder une certaine rigidité pour éviter que l'application d'un couple de dévissage élevé ne permettent de passer outre le blocage en rotation du bouchon par les ergots 24 et les saillies 48.

A ces effets, le récipient 4, monobloc, est réalisé dans un matériau ou un mélange de matériaux possédant un module d'Young E1 inférieur au module d'Young E2 du matériau ou du mélange de matériaux constituant le bouchon 8, également monobloc.

Les matériaux suivants sont compatibles avec un usage médical, et sont utilisables pour la réalisation du récipient 4 et du bouchon 8 : le polyéthylène basse densité (PEBD), le polyéthylène moyenne densité (PEMD), le polyéthylène haute densité (PEHD), le polypropylène et le polyethylène-téréphtalate (PET).

Le PEBD possède une densité comprise entre 0.920 et 0.930. Le PEHD possède une densité comprise entre 0.940 et 0.960. Le PEMD possède une densité comprise entre 0.930 et 0.940.

Le PEBD et le PEHD sont particulièrement avantageux par leur coût de revient faible. En outre, le PEBD, le PEMD et le PEHD se mélangent facilement et sont utilisable dans des procédés de fabrication par injection-soufflage avantageux pour la réalisation de récipients en ces matériaux.

Le PEBD possède un module d'Young inférieur à celui du PEMD, lui-même inférieur à celui du PEHD. Un mélange possédera un module d'Young intermédiaire en fonction de la teneur relative du mélange en PEBD, PEMD et PEHD.

Ainsi, avantageusement, on réalise le récipient 4 en PEBD ou en mélange de PEBD avec du PEMD et/ou du PEHD, et le bouchon 8 en mélange de PEBD avec du PEMD et/ou du PEHD, en PEMD ou en mélange de PEMD et de PEHD, ou en PEHD.

De préférence, pour obtenir une rigidité du bouchon 8 suffisante, le mélange constituant le bouchon 8 possède au moins 20% de PEHD en masse.

Dans un mode de réalisation particulier, le récipient 4 est en PEBD uniquement, et le bouchon 8 est réalisé à partir d'un mélange de PEBD et de PEHD.

Le col 14 possède un diamètre réduit par rapport à celui de la jupe 40, de sorte que les ergots 24 possèdent une longueur L importante. En outre, chaque ergot 24 s'amincit à son extrémité extérieure du fait de la prévision d'une surface de glissement 28.

Afin d'assurer une rigidité en flexion suffisante de chaque ergot 24, en particulier à son extrémité extérieure, chaque ergot 24 possède une surface de transition 31 sensiblement plane s'étendant entre sa surface de glissement 28 et sa surface de blocage 30

Ainsi, chaque ergot 24 possède, sur toute sa longueur L (figure 4), une épaisseur e (figure 4) minimale, supérieure de préférence à 0.5 mm.

En outre, la surface de glissement 28 prend la forme d'un chanfrein entre une face de l'ergot 24 opposée à la surface de blocage 30 et la surface de transition 31. Une telle surface de glissement 28 assure un glissement suffisant, en limitant la diminution de la rigidité en flexion de la portion d'extrémité extérieure de l'ergot 24.

Par ailleurs, du fait que chaque ergot 24 s'appuie sur la couronne 31 a, chaque ergot 24 est rigidifié.

L'ensemble 2 conforme à l'invention permet d'éviter que le bouchon 8 ne soit ouvert en exerçant seulement un couple de dévissage du bouchon 8. Ceci limite le risque qu'un enfant réussisse à ouvrir l'ensemble 2.

L'ensemble 2 possède également un témoin de première utilisation, qui assure la sécurité d'utilisation de l'ensemble 2.

Les deux jupes 38 et 40 indépendantes permettent une fabrication simple et à faible coût du bouchon 8 en intégrant sur celui-ci les différentes fonctions (vissage, sécurité, témoin de première utilisation).

L'invention s'applique également aux ensembles de conditionnement et de distribution d'autres liquides médicaux, prévus pour une distribution goutte par goutte, tels que les solutions pour le nez ou les oreilles.

## Revendications

1. Ensemble de conditionnement et de distribution de collyre, du type comprenant :
- un récipient (4) destiné à contenir du liquide, le récipient (4) possédant un col (14) ;
- un embout (6) compte-gouttes de distribution disposé à l'extrémité du col (14), l'embout (6) et le récipient (4) permettant la distribution de liquide goutte par goutte par pincement du récipient (4) entre les doigts d'une main ;
- un bouchon (8) de fermeture du récipient (4) prévu pour être vissé sur le col (14) ; et
- un témoin de première ouverture sous la forme d'une ceinture d'inviolabilité (10) reliée au bouchon (8) par une région frangible (52) destinée à se rompre lors de la première ouverture du bouchon (8) ;
**caractérisé en ce que** le bouchon (8) et le col (14) possèdent des moyens de sécurité interdisant le dévissage du bouchon (8) par la seule application d'un couple de dévissage, et autorisant le dévissage du bouchon (8) lorsqu'une action supplémentaire est exercée conjointement au couple de dévissage du bouchon (8).

2. Ensemble selon la revendication 1, **caractérisé en ce que** le récipient (4) étant réalisé dans un matériau ou un mélange de matériaux possédant un module d'Young (E1) inférieur au module d'Young (E2) du matériau ou du mélange de matériaux dans lequel est réalisé le bouchon (8).

3. Ensemble selon la revendication 1 ou 2, **caractérisé en ce que** le récipient (4) et le bouchon (8) sont chacun réalisé à partir d'un matériau ou d'un mélange de matériaux choisi(s) parmi les matériaux suivants : le polyéthylène basse densité (PEBD), le polyéthylène moyenne densité (PEMD), le polyéthylène haute densité (PEHD), le polypropylène et le polyethylène-téréphtalate (PET).

4. Ensemble selon l'une quelconque des revendications précédentes, **caractérisé en ce que** le récipient (4) et le bouchon (8) sont réalisés à partir de polyéthylène basse densité (PEBD) et de polyéthylène haute densité (PEHD), le bouchon (8) possédant une proportion en masse de polyéthylène haute densité (PEHD) plus importante que le récipient (4).

5. Ensemble selon la revendication 4, **caractérisé en ce que** le récipient (4) est constitué uniquement de polyéthylène basse densité (PEBD), et **en ce que** le bouchon (8) comprend au moins 20% en masse de polyéthylène haute densité (PEHD), le reste étant constitué de polyethylène basse densité (PEBD).

6. Ensemble selon l'une quelconque des revendications précédentes, **caractérisé en ce que** le récipient (4) possède une contenance inférieure à 30 ml, de préférence inférieure à 20 ml.

7. Ensemble selon l'une quelconque des revendications précédentes, **caractérisé en ce que** les moyens de sécurité permettent le dévissage du bouchon (8) si le bouchon (8) est pincé pendant le dévissage.

8. Ensemble selon l'une quelconque des revendications précédentes, **caractérisé en ce que** les moyens de sécurité possèdent des moyens (48, 24) de blocage unidirectionnel en rotation du bouchon (8) par rapport au col (14), les moyens de blocage unidirectionnel étant débrayables par pincement du bouchon (8).

9. Ensemble selon la revendication 8, **caractérisé en ce que** le bouchon (8) possède une première jupe (40) externe coaxiale à et entourant une deuxième jupe (38) portant un filetage (44) de vissage du bouchon (8) sur le col (14), les moyens de blocage possédant au moins un relief intérieur (48) de la première jupe (40) et au moins un relief extérieur (24) du col (14) complémentaires, pouvant être dégagés l'un de l'autre par pincement de la première jupe (40) sur deux zones (51) décalées angulairement par rapport au ou à chaque relief intérieur (48).

10. Ensemble selon la revendication 9, **caractérisé en ce que** les moyens de blocage possèdent deux reliefs intérieurs (48) disposés sur la jupe (40), diamétralement opposés et décalés angulairement de 90° par rapport aux zones d'appui (51), le col (14) comprenant deux reliefs extérieurs (24) diamétralement opposés.

11. Ensemble selon la revendication 9 ou 10, **caractérisé en ce que** le ou chaque relief extérieur du col (14) est formé par un ergot (24) en saillie radialement vers l'extérieur du col (14) l'ergot (24) possédant une surface de blocage (30) radiale, une surface de glissement (28) inclinée dans son ensemble par rapport à la surface de blocage (30), et une surface de transition (31) s'étendant entre la surface de blocage (30) et la surface de glissement (28), la surface de transition (31) étant sensiblement perpendiculaire à la surface de blocage (30).

12. Ensemble selon l'une quelconque des revendications 9 à 10, **caractérisé en ce que** le col (14) possède une couronne (31 a) annulaire de renfort s'étendant en saillie vers l'extérieur du col (14) et passant par les reliefs extérieurs (24) pour les rigidifier.

13. Ensemble selon l'une quelconque des revendication précédentes, **caractérisé en ce que** le bouchon (8) possède un organe (46) de fermeture étanche de l'embout (6), obturant l'embout (6) au moins sur la course du bouchon (8) pendant laquelle les moyens de sécurité sont opérationnels pour assurer l'étanchéité et la stérilité à l'intérieur du récipient (4).

14. Ensemble selon l'une quelconque des revendications précédentes, **caractérisé en ce que** le récipient contient un collyre.

## Claims

1. Assembly for holding and dispensing a medical liquid, of the type comprising:
- a vessel (4) for containing the liquid, the vessel (4) having a neck (14);
- a dispensing dropper end fitting (6) disposed at the end of the neck (14), the end fitting (6) and the vessel (4) allowing the liquid to be dispensed drop by drop by squeezing the vessel (4) between the fingers of one hand; and
- a cap (8) for closing the vessel (4), provided to be screwed onto the neck (14);
- a first-opening indicator in the form of a tamper-evident band (10) connected to the cap (8) by a brittle region (52) which breaks when the cap (8) is first opened;
**characterized in that** the cap (8) and the neck (14) have safety means preventing the cap (8) from being unscrewed only by applying an unscrewing torque, and allowing the cap (8) to be unscrewed when an additional action is applied together with the torque for unscrewing the cap (8).

2. Assembly according to claim 1, **characterized in that** the vessel (4) is made of a material or mixture of materials having a Young's modulus (E1) less than the Young's modulus (E2) of the material or mixture of materials of which the cap (8) is made.

3. Assembly according to either claim 1 or claim 2, **characterized in that** the vessel (4) and the cap (8) are each made from a material or mixture of materials selected from the following materials: low-density polyethylene (LDPE), medium-density polyethylene (MDPE), high-density polyethylene (HDPE), polypropylene and polyethylene terephthalate (PET).

4. Assembly according to any one of the preceding claims, **characterized in that** the vessel (4) and the cap (8) are made from low-density polyethylene (LDPE) and high-density polyethylene (BDPE), the cap (8) having a greater proportion by mass of high-density polyethylene (HDPE) than the vessel (4).

5. Assembly according to claim 4, **characterized in that** the vessel (4) consists purely of low-density polyethylene (LDPE), and **in that** the cap (8) comprises at least 20% by mass of high-density polyethylene (HDPE), the remainder consisting of low-density polyethylene (LDPE).

6. Assembly according to any one of the preceding claims, **characterized in that** the vessel (4) has a capacity of less than 30 ml, preferably less than 20 ml.

7. Assembly according to any one of the preceding claims, **characterized in that** the safety means make it possible to unscrew the cap (8) if the cap (8) is squeezed during the unscrewing.

8. Assembly according to any one of the preceding claims, **characterized in that** the safety means have means (48, 24) for the unidirectional blocking of the rotation of the cap (8) relative to the neck (14), wherein it is possible to disengage the unidirectional blocking means by squeezing the cap (8).

9. Assembly according to claim 8, **characterized in that** the cap (8) has a first external skirt (40), coaxial with and enclosing a second skirt (38) bearing a thread (44) for screwing the cap (8) onto the neck (14), the blocking means having at least one internal feature (48) on the first skirt (40) and at least one external feature (24) on the neck (14), which features are complementary and can be disengaged from one another by squeezing the first skirt (40) in two regions (51) which are angularly offset relative to each internal feature (48).

10. Assembly according to claim 9, **characterized in that** the blocking means have two diametrically opposite internal features (48), disposed on the skirt (40) and angularly offset by 90° relative to the pressing regions (51), the neck (14) comprising two diametrically opposite external features (24).

11. Assembly according to either claim 9 or claim 10, **characterized in that** each external feature of the neck (14) is formed by a lug (24) projecting radically towards the outside of the neck (14), the lug (24) having a radial blocking surface (30), a sliding surface (28) inclined as a whole relative to the blocking surface (30), and a transition surface (31) extending between the blocking surface (30) and the sliding surface (28), the transition surface (31) being substantially perpendicular to the blocking surface (30).

12. Assembly according to any one of claims 9 to 10, **characterized in that** the neck (14) has an annular reinforcing rim (31a) extending so as to project towards the outside of the neck (14) and passing through the external features (24) so as to rigidify them.

13. Assembly according to any one of the preceding claims, **characterized in that** the cap (8) has an element (46) for closing the end fitting (6) tightly, sealing the end fitting (6) at least over the part of the path of the cap (8) during which the safety means operate to ensure the tightness and sterility of the interior of the vessel (4).

14. Assembly according to any one of the preceding claims, **characterized in that** the vessel contains an ophthalmic medical liquid of the collyrium type.

## Patentansprüche

1. Anordnung zum Verpacken und zur Ausgabe von Augentropfen, derart, dass sie umfasst:
- einen Behälter (4), der vorgesehen ist, eine Flüssigkeit aufzunehmen, wobei der Behälter (4) einen Hals (14) besitzt;
- einen Tropferansatz (6) für die Ausgabe, der am Ende des Halses (14) angeordnet ist, wobei der Ansatz (6) und der Behälter (4) die Verteilung der Flüssigkeit tröpfchenweise durch Zusammendrücken des Behälters (4) zwischen den Fingern einer Hand gestatten;
- einen Stopfen (8) zum Verschließen des Behälters (4), der vorgesehen ist, auf den Hals (14) geschraubt zu werden; und
- eine Anzeige für eine erste Öffnung in Form eines Streifens (10) der Unverletzbarkeit, der mit dem Stopfen (8) durch einen zerstörbaren Bereich (52) verbunden ist, der dazu dient, bei der ersten Öffnung des Stopfens (8) zu zerbrechen;
**dadurch gekennzeichnet, dass** der Stopfen (8) und der Hals (14) Sicherheitsmittel besitzen, die das Abschrauben des Stopfens (8) durch die einzige Anwendung eines Abschraubmoments verwehren und das Abschrauben des Stopfens (8) zulassen, wenn eine zusätzliche Einwirkung zusammen mit dem Abschraubmoment des Stopfens (8) ausgeübt wird.

2. Anordnung nach Anspruch 1, **dadurch gekennzeichnet, dass** der Behälter (4) aus einem Material oder einem Materialgemisch hergestellt ist, das einen Elastizitätsmodul (E1) besitzt, der kleiner ist als der Elastizitätsmodul (E2) des Materials oder des Materialgemischs, aus dem der Stopfen (8) hergestellt ist.

3. Anordnung nach Anspruch 1 oder 2, **dadurch gekennzeichnet, dass** der Behälter (4) und der Stopfen (8) jeweils aus einem Material oder einem Materialgemisch hergestellt ist, das aus den folgenden Materialien ausgewählt ist:
Polyethylen niedriger Dichte (PEBD), Polyethylen mittlerer Dichte (PEMD), Polyethylen hoher Dichte (PEHD), Polypropylen und Polypropylenterephtalat (PET).

4. Anordnung nach einem beliebigen der vorhergehenden Ansprüche, **dadurch gekennzeichnet, dass** der Behälter (4) und der Stopfen (8) aus einem Polyethylen niedriger Dichte (PEBD) und aus Polyethylen hoher Dichte (PEHD) hergestellt sind, wobei der Stopfen (8) ein größeres Massenverhältnis an Polyethylen hoher Dichte (PEHD) besitzt als der Behälter (4).

5. Anordnung nach Anspruch 4, **dadurch gekennzeichnet, dass** der Behälter (4) nur aus Polyethylen niedriger Dichte (PEBD) besteht und dass der Stopfen (8) mindestens 20% bezüglich der Masse an Polyethylen hoher Dichte (PEHD) umfasst, wobei der Rest aus Polyethylen niedriger Dichte (PEBD) besteht.

6. Anordnung nach einem beliebigen der vorhergehenden Ansprüche, **dadurch gekennzeichnet, dass** der Behälter (4) ein Fassungsvermögen kleiner als 30 ml, vorzugsweise kleiner als 20 ml besitzt.

7. Anordnung nach einem beliebigen der vorhergehenden Ansprüche, **dadurch gekennzeichnet, dass** die Sicherheitsmittel das Abschrauben des Stopfens (8) gestatten, wenn der Stopfen (8) während des Abschraubens zusammengeklemmt wird.

8. Anordnung nach einem beliebigen der vorhergehenden Ansprüche, **dadurch gekennzeichnet, dass** die Sicherheitsmittel Mittel (48, 24) zum Blockieren der Drehung des Stopfens (8) in eine Richtung in Bezug auf den Hals (14) besitzen, wobei die Mittel zum Blockieren in eine Richtung durch Zusammenklemmen des Stopfens (8) ausschaltbar sind.

9. Anordnung nach Anspruch 8, **dadurch gekennzeichnet, dass** der Stopfen (8) eine erste äußere Schürze (40) besitzt, die koaxial zu einer zweiten Schürze (38) ist und diese umgibt und die ein Innengewinde (44) zum Schrauben des Stopfens (8) auf den Hals (14) trägt, wobei die Blockiermittel mindestens ein Innenrelief (48) der ersten Schürze (40) und mindestens ein komplementäres Außenrelief (24) des Halses (14) besitzen, die voneinander gelöst werden können durch Drücken der ersten Schürze (40) an zwei Zonen (51), die in Bezug auf das oder jedes Innenrelief (48) winkelmäßig versetzt sind.

10. Anordnung nach Anspruch 9, **dadurch gekennzeichnet, dass** die Blockiermittel zwei Innenreliefs (48) besitzen, die auf der Schürze (40) diametral entgegengesetzt und winkelmäßig um 90° in Bezug auf die Auflagezonen (51) angeordnet sind, wobei der Hals (14) zwei diametral entgegengesetzte Außenreliefs (24) umfasst.

11. Anordnung nach Anspruch 9 oder 10, **dadurch gekennzeichnet, dass** das oder jedes Außenrelief des Halses (14) durch einen Nocken (24) gebildet wird, der radial nach außen vom Hals (14) hervorspringt, wobei der Nocken (24) eine radiale Blockierfläche (30), eine in ihrer Gesamtheit in Bezug auf die Blockierfläche (40) geneigte Gleitfläche (28) und eine Übergangsfläche (31) besitzt, die sich zwischen der Blockierfläche (30) und der Gleitfläche (28) erstreckt, wobei sich die Übergangsfläche (31) im Wesentlichen senkrecht zur Blockierfläche (30) befindet.

12. Anordnung nach einem beliebigen der Ansprüche 9 bis 10, **dadurch gekennzeichnet, dass** der Hals (14) einen ringförmigen Verstärkungskranz (31a) besitzt, der sich hervorspringend vom Hals (14) nach außen erstreckt und durch die Außenreliefs (24) für ihre Versteifung hindurchgeht.

13. Anordnung nach einem beliebigen der vorhergehenden Ansprüche, **dadurch gekennzeichnet, dass** der Stopfen (8) ein Organ (46) zum dichten Verschließen des Ansatzes (6) besitzt, das den Ansatz (6) mindestens über den Hub des Stopfens (8) verschließt, während dem die Sicherheitsmittel betätigbar sind, um die Dichtigkeit und die Sterilität im Innern des Behälters (4) sicherzustellen.

14. Anordnung nach einem beliebigen der vorhergehenden Ansprüche, **dadurch gekennzeichnet, dass** der Behälter ein Augenwasser enthält.
